# EUROPEAN PATENT APPLICATION

(11) **EP 3 725 296 A1**
(43) Date of publication of application: **21.10.2020**
(21) Application number: 18889921.5
(22) Date of filing: 13.12.2018
(51) Int. Cl.: A61K 8/98, A61K 35/17, A61Q 19/00, A61Q 19/08, A61Q 7/00

(54) **COSMETIC COMPOSITION AND PHARMACEUTICAL COMPOSITION FOR ALLEVIATING ATOPIC DERMATITIS, HAIR LOSS, AND WOUNDS OR REDUCING SKIN WRINKLES**

(30) Priority: 14.12.2017 KR 20170172483
(71) Applicant: Green Cross WellBeing Corporation, Seongnam-si, Gyeonggi-do, 13595 (KR)
(72) Inventor: OH, Chang Taek, Seongnam-si, Gyeonggi-do 13595 (KR); LIM, Min Ju, Seongnam-si, Gyeonggi-do 13595 (KR); KIM, Jeom Yong, Seongnam-si, Gyeonggi-do 13595 (KR); PARK, Sun Kyu, Seongnam-si, Gyeonggi-do 13595 (KR); LEE, Jong Hun, Seongnam-si, Gyeonggi-do 13595 (KR); JANG, Min Jung, Seongnam-si, Gyeonggi-do 13595 (KR); KIM, Su Ae, Seongnam-si, Gyeonggi-do 13595 (KR); LIM, Su Hwan, Seongnam-si, Gyeonggi-do 13595 (KR); KIM, Beom Joon, Seoul 06974 (KR); BAK, Dong Ho, Seoul 06974 (KR); KWON, Tae Rin, Seoul 06974 (KR); NA, Jung Tae, Seoul 06974 (KR)
(74) Representative: dompatent von Kreisler Selting Werner - Partnerschaft von Patent- und Rechtsanwälten mbB
(86) International application number: PCT/KR2018/015806
(87) International publication number: WO 2019/117633

(57) **Abstract**

The present invention provides a cosmetic composition and a pharmaceutical composition comprising secretions of natural killer cells, which are excellent in increasing various factors related to improvement of atopic dermatitis, hair loss, wounds or skin wrinkles, and in enhancing an expression level, and thereby having excellent alleviation effects on the symptoms.

## Description

### [Technical Field]

The present invention relates to a cosmetic composition and a pharmaceutical composition for improving atopic dermatitis, hair loss, wounds or skin wrinkles.

### [Background Art]

Among cells to form the immune system, in particular, natural killer cells (NK cells) are cells acting at the front of *in vivo* immune system defense mechanism, such that function to remove tumor cells, bacteria, intercellular parasites or virus-infected host cells without pre-sensitization by an antigen, reject inadequate marrow transplantation, and regulate immune responses of T cells.

Among such various functions of NK cells, specifically, since ability thereof to selectively kill cancer cells has been discovered, a number of related researches were performed. In the case of a cancer patient, the number or activity of NK cells in the peripheral blood were decreased, compared to those in normal subjects. In animal experiments, it was found that a risk of developing cancer and metastasis of cancer is increasing with a reduction in NK cell activity.

Further, NK cells play an important role in the innate immune response against host-infected pathogens or cancer cells, as well as the acquired immune response through cytokine secretion. Herein, a major mechanism using NK cells to kill target cells is to secrete cell-lytic granules such as perforin and granzyme B into the target cells through immune synapses. The secreted perforin makes a hole in a wall of the target cell and granzyme B entered the target cell through the hole may induce apoptosis of the target cell.

As such, it was found that NK cells play a crucial role in oncogenesis wherein cells are transformed into malignant cells and in a defense mechanism in the early stage of viral infection. However, studies on the secreted substances (that is, secretions) other than the NK cells themselves, new use thereof, etc. are still not sufficient.

### [Summary of Invention]

### [Problems to be Solved by Invention]

It is an object of the present invention to provide a cosmetic composition for improving atopic dermatitis, hair loss, wounds or skin wrinkles.

In addition, another object of the present invention is to provide a pharmaceutical composition for preventing or treating atopic dermatitis, hair loss, wounds or skin wrinkles.

### [Means for Solving Problems]

1. A cosmetic composition for improving atopic dermatitis, hair loss, wounds or skin wrinkles, comprising secretions of natural killer cells.
2. The composition according to above 1, wherein the natural killer cell secretion includes at least one selected from the group consisting of IL-2, TIMP2, VEGF, PDGF-AA and IL-10.
3. The composition according to above 1, wherein the natural killer cell secretion is obtained from a residual culture liquid remaining after culturing natural killer cells and then separating and purifying the same.
4. The composition according to above 3, wherein the culturing includes culturing seed cells in a medium containing feeder cells, a feeder cell stimulating factor and a growth factor.
5. The composition according to above 4, wherein the seed cells are human-derived cells with a potential to differentiate into natural killer cells.
6. The composition according to above 5, wherein the human-derived cell is selected from the group consisting of peripheral blood, peripheral blood leukocyte cells, peripheral blood mononuclear cells (PBMC), enriched natural killer cells, isolated natural killer cells, cord blood, hematopoietic stem cells and induced pluripotent stem cells.
7. The composition according to above 4, wherein the growth factor is interleukin.
8. A pharmaceutical composition for improving atopic dermatitis, hair loss, wounds or skin wrinkles, comprising secretions of natural killer cells.
9. The composition according to above 8, wherein the natural killer cell secretion includes at least one selected from the group consisting of IL-2, TIMP2, VEGF, PDGF-AA and IL-10.
10. The composition according to above 8, wherein the natural killer cell secretion is obtained from a residual culture liquid remaining after culturing natural killer cells and then separating and purifying the same.
11. The composition according to above 10, wherein the culturing includes culturing seed cells in a medium containing feeder cells, a feeder cell stimulating factor and a growth factor.
12. The composition according to above 11, wherein the seed cells are human-derived cells with a potential to differentiate into natural killer cells.
13. The composition according to above 12, wherein the human-derived cell is selected from the group consisting of peripheral blood, peripheral blood leukocyte cells, peripheral blood mononuclear cells (PBMC), enriched natural killer cells, isolated natural killer cells, cord blood, hematopoietic stem cells and induced pluripotent stem cells.
14. The composition according to above 11, wherein the growth factor is interleukin.

### [Advantageous Effects]

According to the present invention, the secretion is excellent in increasing various factors related to improvement of atopic dermatitis, hair loss, wounds or skin wrinkles, and in enhancing an expression level. Thereby, the cosmetic composition of the present invention including the secretion is excellent in improving the symptoms described above, and the pharmaceutical composition is excellent in prevention or treatment of the above diseases.

### [Brief Description of Drawings]

FIG. 1 is a graph illustrating results of evaluating a difference in fibroblast proliferative ability between NK-CM and NK medium.
FIG. 2 is diagrams illustrating results of evaluating cell migration ability and cell proliferative ability in regard to HDF-N cells of NK-CM.
FIG. 3 is diagrams illustrating results of evaluating collagen production ability of NK-CM.
FIG. 4 is a graph illustrating results of monitoring cell viability in regard to keratinocytes by NK-CM treatment.
FIG. 5 is a graph illustrating results of monitoring cell viability in regard to outer root sheath (ROS) cells by NK-CM treatment.
FIG. 6 is graphs illustrating relationships between expression of proinflammatory cytokines in HaCaT cells and NK-CM.
FIG. 7 is graphs illustrating relationships between expression of proinflammatory cytokines in HMC-1 cells and NK-CM.
FIG. 8 is diagrams illustrating results of component analysis of NK conditioned-medium (NK-CM).

### [Mode for Carrying out Invention]

Hereinafter, the present invention will be described in detail.

The cosmetic composition of the present invention for improving atopic dermatitis, hair loss, wounds or skin wrinkles may include secretions of natural killer cells.

Natural killer cells (NK cells) are lymphoid cells playing a role in immune response, which are present in about 10 to 15% of normal blood and have high killing ability when they react with non-self antigen. In generation of cells infected with various viruses, bacterial infiltration or generation of abnormal cells, NK cells react non-specifically and immediately to remove foreign matters. Therefore, natural killer cells have been studied for application as immuno-anticancer agents.

However, the inventors have found that secretions secreted by the natural killer cells other than the natural killer cells themselves have effects on atopic dermatitis, hair loss, wounds, and skin wrinkles, which are different from the above applications, and the present invention has been designed and completed on the basis of the above finding.

The secretions of the natural killer cells according to the present invention may include at least one selected from the group consisting of interleukin-2 (IL-2), metallopeptidase inhibitor 2 (TIMP2), vascular endothelial growth factor (VEGF), platelet-derived growth factor-AA (PDGF-AA) and interleukin-10 (IL-10).

Among the above components contained in the secretions of the natural killer cells according to the present invention, IL-2 and TIMP2 are factors associated with wrinkle improvement, VEGF and PDGF-AA are factors associated with hair loss improvement, and IL-2 and IL-10 are factors associated with atopic dermatitis improvement.

The secretions of natural killer cells according to the present invention may be obtained, for example, from a residual culture liquid remaining after culturing natural killer cells and then separating and purifying the same, or the residual culture liquid may be directly used, but it is not limited thereto.

Hereinafter, the animal cell culture medium and the following components may include components that generally help growth of cells or normally stabilize cells, and that influence the growth of cells. Although a degree of cell growth may vary depending on these components, secretive components of the cells are not affected thereby. The following example is merely one embodiment, and it is not limited thereto.

Natural killer cells may be cultured using a conventional animal cell culture medium known in the art without limitation thereof, and may include, for example, CellGro medium (Cellgenix), AIM-V medium, RPMI1640 medium, XVIVO 20, RPMI1640 and the like.

Culturing of natural killer cells may include culturing seed cells to obtain natural killer cells or culturing the natural killer cells.

As used herein, the term "seed cell" means a cell capable of increasing into a natural killer cell through proper culture, and specifically, it may be a human-derived cell with a potential to differentiate into a natural killer cell. The cell used herein may include at least one selected from, for example, the group consisting of peripheral blood, peripheral blood leukocyte, peripheral blood mononuclear cell (PBMC), enriched natural killer cell, isolated natural killer cell, cord blood, hematopoietic stem cell and induced pluripotent stem cell, but it is not limited thereto.

Culturing of the seed cells may be performed in a medium supplemented with components for promoting the culture of natural killer cells.

The components for promoting the culture may include, for example, feeder cells, feeder cell stimulating factors, growth factors and the like.

The feeder cell is a cell that does not allow to be divided and proliferated, however, due to metabolic activity, produces a variety of metabolites to help proliferation of natural killer cells.

The feeder cell used herein may be any one so long as it can support the proliferation of natural killer cells without limitation thereof, and may include, for example, a cell line into which a gene has been introduced, a peripheral blood leukocyte (PBL) cell treated with various cytokines or compounds, a peripheral blood leukocyte (PBL), T-cell, B-cell, monocyte, and CD4(+) T cell of or the like. For specific examples, at least one of the self-peripheral blood leukocyte (PBL) and CD4(+) T cells may be used, but it is not limited thereto.

Using the feeder cells in an inactivated state, safety may be ensured. As a method for inactivation, a conventional method known in the art may be used. For example, radiation of gamma-rays may be used. Such inactivated feeder cells may include isolated T-cells (or purified T cells).

As the feeder cell stimulating factor, for example, an anti-CD3 antibody may be used.

As used herein, the term "anti-CD3 antibody" means an antibody that binds specifically to CD3 antigen as a molecule group binding to a T cell receptor (TCR) to form an antigen recognition complex, wherein the CD3 molecule binds to TCR and transmits an antigen-recognizing signal into the cell.

The anti-CD3 antibody useable in the present invention is not limited so long as it is an antibody with binding property to CD3, and is preferably selected from the group consisting of OKT3, UCHT1 and HIT3a, without limitation thereof. Anti-CD3 antibodies may be included, for example, in an amount of 0.1 to 100 ng/ml, but it is not limited thereto.

The growth factor may include, for example, cytokine. The cytokine is a growth factor, and preferably, one or more selected from interleukins, but it is not limited thereto.

Interleukin is a generic term indicating proteinaceous and bioactive substances produced by immune-competent cells such as lymphocytes, monocytes and macrophages. Examples of interleukins useable herein may include at least one selected from the group consisting of interleukin-2 (IL-2), interleukin-12 (IL-12), interleukin-15 (IL-15), interleukin-18 (IL-18) and interleukin-21 (IL-21). In particular, IL-2 is preferably used without limitation thereof. Further, those skilled in the art will recognize that other cytokines may also be used without limitation so long as they meet the purpose of the present invention. The cytokine may be included in an amount of, for example, 10 to 2000 U/ml, but it is not limited thereto.

Further, serum or plasma and additional proliferation factors to support proliferation of lymphocytes may be added, followed by culturing. Types of the serum or plasma added to the medium are not particularly limited, but may include any commercially available animal-derived ones, preferably, those derived from humans, and more preferably, the serum or plasma derived from one's own. For example, a combination of cytokines to proliferate lymphocytes from PBMCs, lectins to stimulate lymphocyte proliferation, and the like, may be added, and other methods known to those skilled in the art may also be used.

Further, the medium may further include serum or plasma and an additional proliferation factor supporting the proliferation of lymphocytes, or may include serum or plasma itself. Types of the serum or plasma added to the medium is not particularly limited, but may include any commercially available animal-derived ones, preferably, those derived from humans, and more preferably, the serum or plasma derived from one's own. For example, human AB serum or auto plasma may be used.

With regard to the cosmetic composition including the secretion according to the present invention, components used in a typical cosmetic composition in addition to the above-mentioned active ingredients may be included. For example, conventional supplements such as antioxidants, stabilizers, solubilizing agent, vitamin, pigments and aromatic essences, as well as carriers, may be included.

The cosmetic composition of the present invention may be prepared into any formulation generally produced in the art, and for example, may be formulated in the form of solution, suspension, emulsion, paste, gel, cream, lotion, powder, soap, surfactant-containing cleanser, oil, powder foundation, emulsion foundation, wax foundation, pack, massage cream, spray, etc., but it is not limited thereto. More specifically, the composition may be manufactured into any formulation such as softening beauty wash, nourishing beauty wash, nutritional cream, massage cream, essence, eye cream, cleansing cream, cleansing foam, cleansing water, pack, spray or powder.

When the formulation of the present invention is the paste, cream or gel, an animal oil, vegetable oil, wax, paraffin, starch, tragacanth, cellulose derivative, polyethylene glycol, silicone, bentonite, silica, talc, zinc oxide or the like may be used as a carrier component.

When the formulation of the present invention is the solution or emulsion, a solvent, solubilizing agent or emulsifying agent is used as the carrier component. For example, water, ethanol, isopropanol, ethyl carbonate, ethyl acetate, benzyl alcohol, benzyl benzoate, 1,3-butyl glycol oil, glycerol aliphatic ester, polyethylene glycol or sorbitan fatty acid ester may be used.

When the formulation of the present invention is the suspension, liquid diluents such as water, ethanol or propylene glycol, suspending agents such as ethoxylated isostearyl alcohol, polyoxyethylene sorbitol esters and polyoxyethylene sorbitan esters, microcrystalline cellulose, aluminum metahydroxide, bentonite, agar, tragacanth, or the like may be used as the carrier component.

When the formulation of the present invention is the powder or spray, lactose, talc, silica, aluminum hydroxide, calcium silicate or polyamide powder may be used as a carrier component. In particular, in the case of the spray, the composition may further include propellants such as chloro-fluorohydrocarbon, propane/butane or dimethyether.

When the formulation is the surfactant-containing cleanser, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamido betaine, aliphatic alcohol, fatty acid glyceride, fatty acid diethanolamide, vegetable oil, lanolin derivative, ethoxylated glycerol fatty acid ester, or the like may be used as the carrier component, but it is not limited thereto.

When the cosmetic composition of the present invention is the soap, surfactant-containing cleansing formulation or surfactant-free cleansing formulation, it may be applied to the skin and then wiped off, peeled off or washed with water. Specifically, the soap may be a liquid soap, powdered soap, solid soap or oil soap; the surfactant-containing cleansing formulation may be a cleansing foam, cleansing water, cleansing towel and cleanser pack; and the surfactant-free cleansing formulation may be a cleansing cream, cleansing lotion, cleansing water and cleansing gel, but it is not limited thereto.

In addition, the present invention provides a pharmaceutical composition for improving atopic dermatitis, hair loss, wounds or skin wrinkles, which includes secretions of natural killer cells.

The secretions of natural killer cells may be within the above-described range.

The pharmaceutical composition of the present invention may be formulated into pharmaceutical preparations for preventing and treating degenerative neurological diseases, which includes a pharmaceutically acceptable carrier, diluent or excipient.

The carrier, excipient and diluent may include, for example, lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate and mineral oil.

The pharmaceutical composition of the present invention may be manufactured into an oral formulation such as powder, granule, tablet, capsule, suspension, emulsion, syrup, aerosol, etc., an external preparation, a suppository or a sterilized injection solution type formulation according to conventional methods. Generally, in the case of formulation, diluents or excipients such as fillers, extenders, binders, humectants, disintegrants, surfactants and the like may be usually used to prepare the formulation. The solid formulation for oral administration may include, for example, tablets, pills, powders, granules, capsules and the like. The solid formulation may be prepared by admixing the pharmaceutical composition with at least one excipient, for example, starch, calcium carbonate, sucrose, lactose, gelatin and the like. Other than simple excipients, lubricants such as magnesium stearate and talc may also be used. Examples of the liquid preparation for oral use may include suspending agents, oral liquids, emulsions, syrups and the like. Other than diluents commonly used in the art such as water and liquid paraffin, various excipients such as wetting agents, sweeteners, fragrances, preservatives, etc. may be used. Formulations for parenteral administration may include sterile aqueous solution, non-aqueous solution, suspending agents, emulsions, freeze-dried preparations, suppositories and the like. Examples of the suspending agents may include propylene glycol, polyethylene glycol, vegetable oil such as olive oil, injectable ester such as ethyl oleate and the like. Examples of the suppository base may include witepsol, macrogol, tween 61, cacao fat, laurin fat, glycerogelatin and the like.

A dosage of the pharmaceutical composition of the present invention may vary depending on the age, gender and body weight of a patient, but is generally in a range of 0.1 to 100 mg/kg, preferably 1 to 30 mg/kg, which may be administered in once a day or several times a day in divided doses. The dosage may also be increased or decreased depending on the administration route, degree of disease, gender, weight, age, health condition, diet, administration time, administration method, excretion rate and the like. Accordingly, the dosage is not intended to limit the scope of the invention in any manner.

The pharmaceutical composition of the present invention may be administered to mammals such as rats, mice, livestock, humans, and the like, in various routes. All modes of administration may be expected, for example, skin application, oral, rectal or intravenous, intramuscular, subcutaneous, intra-uterine or intracerebral injection may be used for administration.

Hereinafter, the present invention will be described in detail with reference to examples.

### EXAMPLE: Obtaining natural killer cell secretion

### 1. Obtaining natural killer cell secretion

### 1-1. Preparation of CD3(-) PBMC seed cells

PBS (phosphate buffered saline, LONZA, 17-516Q) was added to PBMCs taken from a healthy donor at a ratio of 1:1, followed by centrifugation at 1500 rpm and 4 °C for 10 minutes. The PBMC pellets were suspended in MACS running buffer (PBS containing 2% FBS and 2 mM EDTA) at 10×10⁶ cells/mL, and cell count was determined using an automatic cell counter.

To obtain seed cells after depletion of CD3(+) cells, 5-10×10⁷ cells were transferred to 50 mL tube and centrifuged at 1200 rpm and 4 °C for 10 min. 400 to 800 µL of MACS running buffer and 100 to 200 µL of CD3 magnetic beads (Miltenyi Biotech, 130050101) were added to PBMC cells at 5-10×10⁷ cells, followed by reaction at 4 °C for 20 minutes. After washing with more than 10 times MACS running buffer, the product was centrifuged at 1350 rpm and 4 °C for 7 minutes and suspended in the final 1 to 2 mL MACS running buffer. Cells were separated by a CS column (Miltenyi Biotech, 130-041-305) mounted on VarioMACS (Miltenyi Biotech), and the column was washed 3 times to recover the cells. Cell count was determined using an automated cell counter. The cells were suspended in the freezing medium and 1-2×10⁷ cells per vial were frozen and stored in liquid nitrogen.

On the day of culture, frozen CD3(-) PBMCs were thawed in a water bath at 37 °C, transferred to a 50 mL tube and suspended by 10 times in PBS (ACD-A PBS buffer) containing 0.6% ACD (citrate-dextrose solution, Sigma-Aldrich, C3821), 0.2% fetal serum bovine (FBS) and 2 mM EDTA, followed by centrifugation at 1200 rpm and 4 °C for 10 minutes. CD3(-) PBMCs were suspended in CellGro SCGM medium (Cellgenix, 20802-0500) and cell count was determined. CD3(-) PBMC seed cells were suspended in CellGro SCGM medium at 1×10⁶ cells/mL.

### 1-2. Preparation of feeder cells

Peripheral blood PBMC feeder cells were added with PBS (phosphate buffered saline, LONZA, 17-516Q) at a ratio of 1:1 and centrifuged at 1500 rpm and 4 °C for 10 minutes. The PBMC pellets were suspended in MACS running buffer (PBS containing 2% FBS and 2 mM EDTA) at 10×10⁶ cells/mL, and the cell count was determined using an automatic cell counter. PBMC feeder cells were suspended in the freezing medium and 1×10⁸ cells/vial was frozen and stored in liquid nitrogen.

On the day of culture, frozen PBMC feeder cells were thawed in a 37 °C water bath, transferred to a 50 mL tube, suspended in 10-fold ACD-A PBS buffer, and then centrifuged at 1200 rpm and 4 °C for 10 minutes. Cells were counted, then suspended at 5×10⁶ cells/mL in CellGro SCGM medium, and inactivated by irradiation with 2000 cGy in a gamma-ray irradiator.

### 1-3. Culture of natural killer cells and recovery of culture liquid

When culturing natural killer cells, a culture medium was prepared by adding 500 IU/mL of IL-2 (Prolactin Injection, Novartis Korea), 10 ng/mL OKT-3 (eBioscience, 16-0037-85) and 1 to 2% donor plasma to CellGro SCGM medium. On 0th day of culture, CD3(-) PBMC seed cells and gamma-irradiated feeder cells were mixed at a ratio of 1:5 and a cell concentration was adjusted to 2×10⁶ cells/mL based on a total number of cells, followed by stationary culture in an incubator at 37 °C for 4 to 5 days.

On 4 to 5th day of the culture, the same amount of culture medium (Cellgro SCGM + 500 IU IL-2+ 1 vol% donor plasma) was added, and the stationary culture was further conducted for 2 to 3 days. On 7th day of the culture, the cells under culturing in the same manner as 0^{th} day of the culture were inoculated into a feeder cell line at a ratio of 1:5, 500 IU/mL of IL-2, 10 ng/mL of OKT-3 and 1 vol% donor plasma, a cell concentration was adjusted to 1×10⁶ cells/mL based on the total number of cells and re-stimulated, followed by stationary culture for 3 days. Then, cell counts were determined at an interval of 2 to 3 days, and the culture medium was further added so as to reach a concentration of 0.5-1×10⁶ cells/mL and cultured for 20 to 21 days.

On 20 to 21^{st} day of the culture, cells under culture were transferred to a 1L centrifuge tube and centrifuged at 1500 rpm and 4 °C for 10 minutes. After centrifugation, the supernatant was recovered and named NK conditioned-media.

Then, 500 IU/mL of IL-2 (Prolactin Injection, Novartis Korea), 10 ng/ml of OKT-3 (eBioscience, 16-0037-85) and 1 to 2% donor plasma were added to the Cellgro SCGM medium to prepare a culture medium, which was named NK cell medium.

### Experimental Example 1. Assessment of cell proliferative ability of NK-CM

In order to assess a difference in proliferative ability between NK cell medium and NK-CM in human fibroblasts, Cell Counting Kit-8 (CCK-8) was used to determine cell proliferative ability.

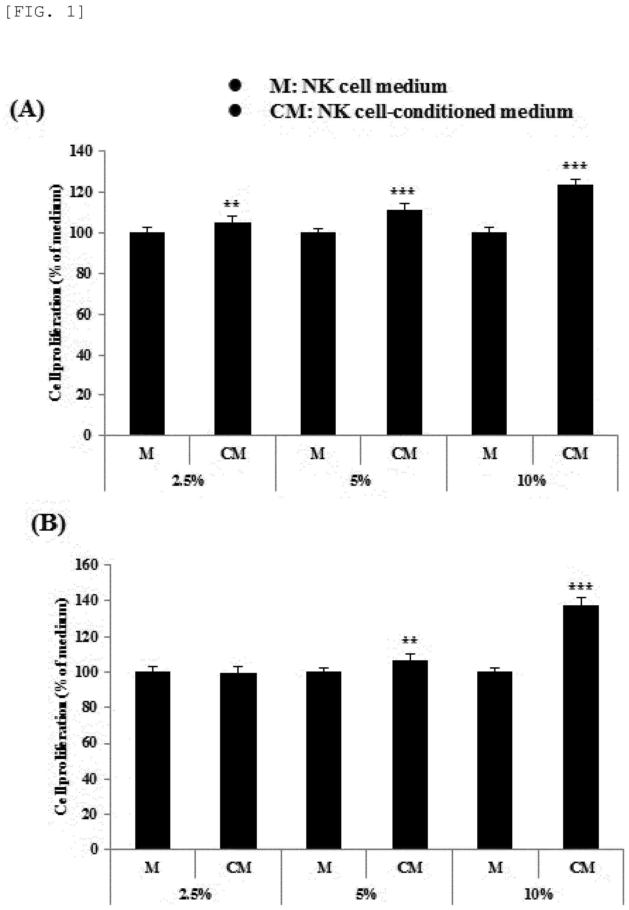

Human fibroblast (HDF-N) cells suspended as single cells were seeded in a 96-well plate at 5×10³ cells/well and cultured at 37 °C. After keeping the cells starved for 24 hours, the cells were treated with NK cell culture medium and NK-CM, respectively, followed by further culture for 24 and 48 hours. In order to determine cell proliferative ability, a Cell Counting Kit-8 reactant was diluted by 1/10 in the supplement-free medium, and then the diluted reactant was applied to the well by 100 µl per each well and reacted for 1 hour. The product was measured by ELISA microplate reader (Model 680, BIO-RAD) with an absorbance at 450 nm. As a positive control, medium supplemented with 10% FBS was used and a cell proliferation rate of each group was calculated as a percentage (%) as compared to the NK cell medium (M). Results thereof are shown in FIG. 1.

As shown in FIG. 1, when comparing NK-CM with NK cell medium at 24 hours, it showed a relatively high proliferative ability at all concentrations, and it was confirmed an increased thereof depending on the concentration. At 48 hours, NK-CM showed no significant difference at 2.5% concentration compared to NK cell culture, but the proliferative ability was increased by 7% at 5% concentration and by maximum 37% at 10% concentration. This result means that NK-CM has higher proliferative effect on the cells than the NK cell medium.

### Experimental Example 2. Assessment of cell proliferative ability of NK-CM

Cell scratch assay was used to assess and determine cell migration and wound restoration abilities of NK-CM in human fibroblasts.

HDF-N cells were seeded in ibidi culture inserts at 1.0×10⁴ cells/inserts on the bottom of a 60 mm dish (µ-Dish 35 mm, High Culture-Inserts; Thistle Scientific Ltd, UK) and cultured for 24 hours under cell culture conditions. After the chamber was removed, the sample was diluted in the medium at a suitable concentration, and the cells were treated with the diluted sample and cultured for 48 hours under the cell culture conditions. 24 and 48 hours later, a migration ability of the cells was photographed through an electron microscope (Eclipse TS100, Nikon Instruments Inc., NY, USA).

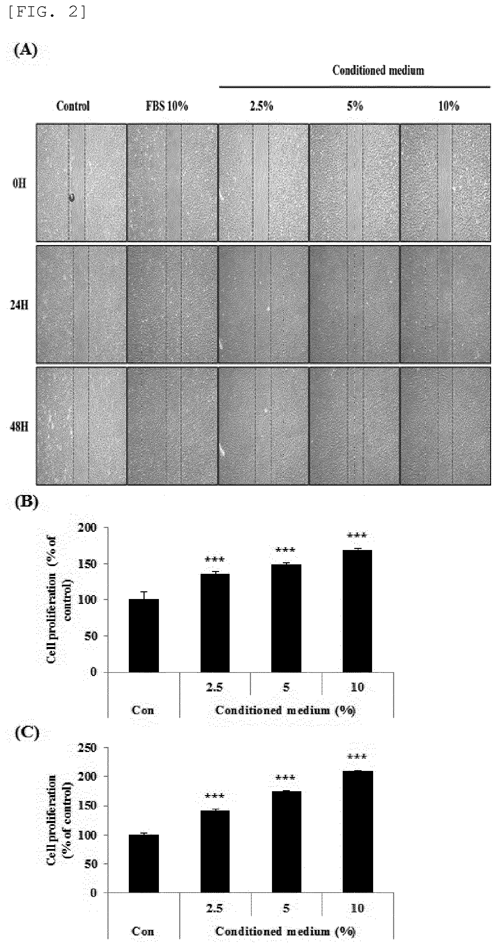

As shown in FIG. 2(A), it could be seen that the cell migration ability and the wound restoration ability were significantly increased as the concentration was increased in the NK-CM treatment group at 24 and 48 hours, as compared to the control.

In order to assess the proliferative ability of NK-CM, HDF-N cells were treated with 2.5, 5, 10%, and then measured at 24 and 48 hours using Cell Counting Kit-8 (CCK-8) .

As shown in FIG. 2 (B, C), it could be seen that, when the NK-CM-treated group and the control were compared at 24 and 48 hours, the cell proliferative ability was increased depending on the concentration. In particular, the maximum concentration of 10% NK-CM increased by 1.6 times at 24 hours and by 2 times at 48 hours, respectively. This result suggests that NK-CM has wrinkle-improving efficacy by increasing the cell migration ability and the wound restoration ability of HDF-N cells, and enhancing the cell proliferative ability.

### Experimental Example 3. Assessment of collagen production ability of NK-CM

In order to assess procollagen synthesis ability of NK-CM, NK-CM was treated to have non-toxic concentrations of 2.5, 5 and 10%, followed by assessment of procollagen synthesis ability at 24 hours.

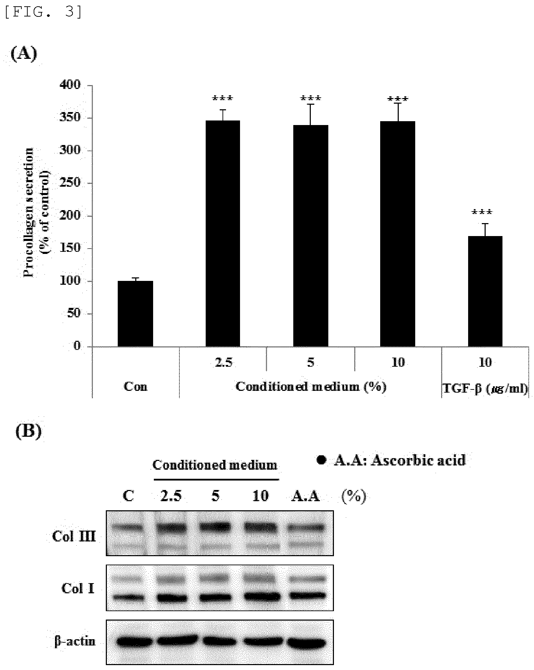

HDF-N cells were seeded in a 24-well plate at a density of 2.0×10⁴ cells/well and then cultured for 24 hours. After the medium was removed and the cells were kept starved for 24 hours, NK-CM was diluted in a medium without media supplement and then applied to the cells. After 24 hours, the culture medium was collected, followed by measuring an amount of procollagen using procollagen type-1 c-peptide (PIP) EIA kit (TAKARA, MK101). Cells deposited to the bottom were dissolved in 1 N NaOH to measure a total protein content, and a procollagen production amount per protein was determined. Each group was calculated as a percentage (%), compared to NK cell culture medium (M), and results thereof are shown in FIG. 3(A).

As shown in FIG. 3(A), when treating TGF-β (10 nM) used as a positive control, procollagen production was increased by 1.7 times, compared to the control. When treating NK culture liquid, procollagen production was increased by about 3.4 times at all concentrations, compared to the control, and statistically significant results were obtained.

A change in expression of collagen protein was determined by western blotting analysis using NK cell culture liquid in human fibroblasts.

HDF-N cells were seeded on a 100 mm plate at 2.0×10⁶ cells and cultured for 24 hours. After the medium was removed and the cells were kept starved for 24 hours, the test substance was diluted in a medium without media supplement and applied to the cells. After culturing for 24 hours, the medium was removed, the cells were washed with DPBS, lysed with a lysis buffer, and centrifuged at 12,000 rpm and 4 °C for 20 minutes to obtain a supernatant, followed by quantification of protein in the supernatant. The quantified protein was subjected to electrophoresis on SDS-PAGE and then a gel protein was blotted with a PVDF membrane. After blocking with 5% BSA, the cells were reacted with a primary antibody at 4 °C for 24 hours, and then, reacted with a secondary antibody for 1 hour. Then, the cells were washed 3 times with a TBS-T buffer for 10 minutes, and a protein expression level was observed using an ECL detection solution. Results thereof are shown in FIG. 3(B).

As shown in FIG. 3(B), expression of Collagen I and III was effectively increased in the NK-CM-treated group, compared to the control. This result suggests that NK-CM induces collagen synthesis and expression of human fibroblasts, thereby offering wrinkle improving efficacy.

### Experimental Example 4. Confirmation of cell viability of keratinocytes by NK-CM treatment

Using Cell Counting Kit-8 (CCK-8) as one of the methods for measuring cell viability, effects of NK-CM on keratinocytes in normal conditions were determined.

HaCaT cells suspended in single cells were seeded in 96-well plates at 1×10⁴ cells/well and cultured in a CO₂ incubator at 37 °C for 24 hours. After culturing, each treatment was performed as follows: on the basis of 100 µL of the cell culture liquid, an added amount of 100% NK-CM was set to a highest concentration and then diluted to a final dilution of 128 times in a 1/2 dilution manner; and HaCaT cells were treated with the diluted NK-CM for 24 hours. CCK-8 solution was then diluted 1:10 with NK-CM-treated HaCaT medium at each concentration and cultured in a CO₂ incubator at 37 °C for 1 hour.

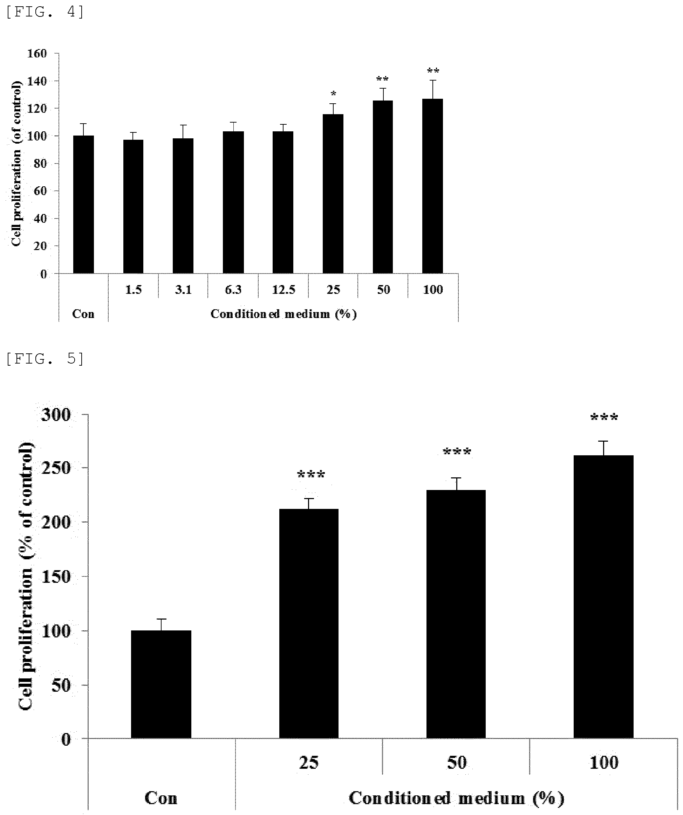

Then, an absorbance at a wavelength of 450 nm was measured using a spectrophotometer. At this time, the reference was set to 450 nm. Cell viabilities of each group were calculated as percentage (%), compared to the control (Con). Results thereof are shown in FIG. 4.

As a result of measuring the death of HaCaT cells by NK-CM, it was confirmed that the cell viability was increased with increasing concentration. This means that even 100% NK-CM had no cytotoxicity to cells, thereby having cell growth effects.

### Experimental Example 5. Confirmation of cell viability of outer root sheath (ORS) by NK-CM treatment

Using Cell Counting Kit-8 (CCK-8) as one of the methods for measuring cell viability, effects of NK-CM on keratinocytes in normal conditions were determined.

ORS cells suspended as single cells were seeded in 96-well plates at 1×10⁴ cells/well and cultured in a CO₂ incubator at 37 °C for 24 hours. After culturing, each treatment was carried out as follows: on the basis of 100 µL of the cell culture liquid, an added amount of 100% NK-CM was set to a highest concentration and then diluted to a final dilution of 4 times in a 1/2 dilution manner; and ORS cells were treated with the diluted NK-CM for 24 hours. Substantially, CCK-8 solution was diluted with NK-CM-treated ORS medium at a ratio of 1:10 at each concentration, and then cultured in a CO₂ incubator at 37 °C for 1 hour.

Then, an absorbance at a wavelength of 450 nm was measured using a spectrophotometer. At this time, the reference was set to 450 nm. Cell viabilities of each group were calculated as percentage (%), compared to the control (Con). Results thereof are shown in FIG. 5.

As a result of measuring the death of ORS cells by NK-CM, it was confirmed that the cell viability was increased with increasing concentration, and even 100% NK-CM had no cytotoxicity to cells. Consequently, this means that NK-CM is helpful for growth of ORS cells as one of major constitutive cells.

### Experimental Example 6. Relationship between proinflammatory cytokines in HaCaT cells and NK-CM treatment

The role of NK-CM in inflammation induction state was confirmed by measuring an expression level of messenger RNA (mRNA) involved in intracellular proinflammatory cytokine secretion through a real time-polymerase chain reaction (RT-PCR).

HaCaT cells were seeded in a 12-well plate at 1×10⁵ cells/well, treated with 50% or 100% NK-CM for 2 hours, and then treated with a recombinant protein TNF-alpha (50 ng/mL) for 24 hours in order to increase expression of proinflammatory cytokines and finally induce inflammatory conditions. After all media containing reagents were washed out, the treated product was mixed with 1 mL TRIzol® RNA separation reagent (Invitrogen, USA). The mixed solution was incubated on ice for 30 minutes, and 400 µL of chloroform 1 was added thereto, followed by reaction for 5 minutes. Next, centrifugation was conducted at 16,000 rpm for 20 minutes. After separating and collecting an upper layer clear solution (that is, supernatant), isopropyl alcohol was added in the same amount as the supernatant and allowed to react for 5 minutes, followed by centrifugation at 16,000 rpm for 10 minutes. The precipitated pellets were washed 3 times with 70% alcohol. Finally, after drying for 4 hours, the product was diluted in 50 µL of purified water to obtain an mRNA sample.

The obtained sample consisted of double stranded DNA (cDNA) synthesized using PrimeScript™ RT master mix (Takara, Japan). 5 µL of 5X PrimeScript™ RT master mix was added to 20 µL of the mRNA sample, and heated at 37 °C for 20 minutes and then at 85 °C for 10 seconds, thereby obtaining a synthesized DNA double helix structure.

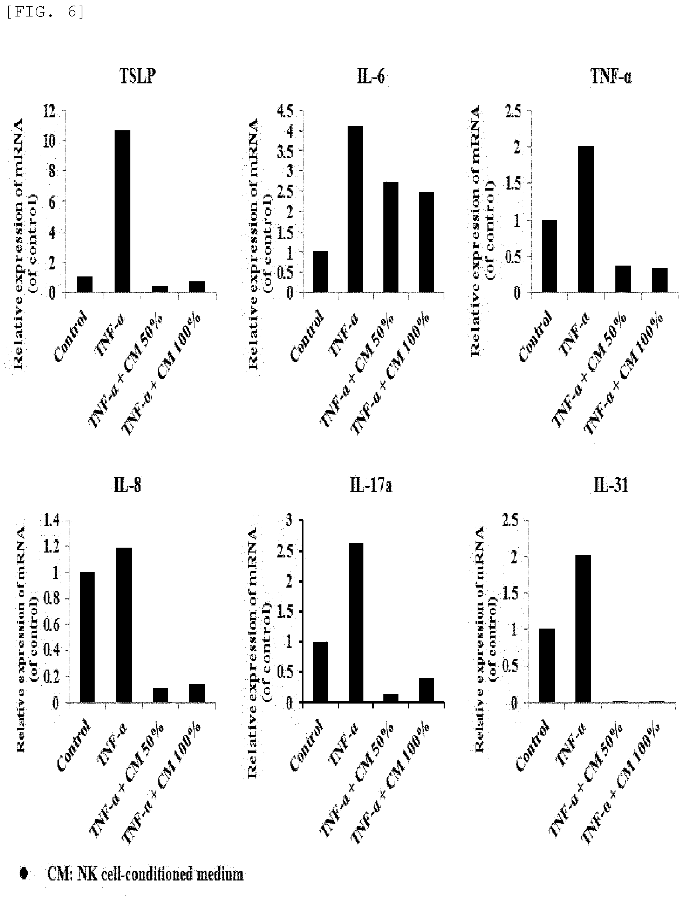

Specific genes were amplified using CFX-96 (Bio-Rad, USA). PCR conditions used for amplification of all genes include: 95 °C for 10 minutes; 95 °C for 30 seconds; and 60 °C for 15 seconds, which were executed by 40 cycles. Expression data were calculated from a cycle threshold (Ct) value using ΔCt according to a quantification method. Further, standardization was conducted using GAPDH. The resultant data were indicated as relative values, compared to the control. Result thereof are shown in FIG. 6.

As shown in FIG. 6, it could be seen that mRNA levels of proinflammatory cytokines such as TSLP, IL-6, TNF-alpha, IL-8, IL-17a and IL-31 were significantly increased in the TNF-alpha treated groups. This means that cell treatment using TNF-alpha may increase the expression of inflammatory cytokines, as compared to a population in normal conditions. However, the results showed that NK-CM significantly reduced the expression of proinflammatory cytokines in the 50% or 100% NK-CM treated group. This suggests that NK-CM may inhibit the expression of proinflammatory cytokines due to TNF-alpha, and consequently, NK-CM has anti-inflammatory effects on HaCaT cells.

### Experimental Example 7. Relationship between expression of proinflammatory cytokine in HMC-1 cells and NK-CM treatment

The role of NK-CM in inflammation conditions was confirmed by measuring an expression level of messenger RNA (mRNA) involved in intracellular proinflammatory cytokine secretion through the real time-polymerase chain reaction (RT-PCR).

Human-derived mast cells, that is, HMC-1 cells were seeded in a 6-well plate at 2×10⁶ cells/well, treated with 50% or 100% NK-CM for 2 hours, treated with PMAC [PMA (25 nM) + A23187 (1 µM)] in order to induce cell degranulation and finally induce inflammatory conditions, and then cultured for 12 hours.

After all media containing reagents were washed out, the treated product was mixed with 1 mL TRIzol® RNA separation reagent (Invitrogen, USA). The mixed solution was incubated on ice for 30 minutes, and 400 µL of chloroform 1 was added thereto, followed by reaction for 5 minutes. Next, centrifugation was conducted at 16,000 rpm for 20 minutes. After separating and collecting an upper layer clear solution (that is, supernatant), isopropyl alcohol was added in the same amount as the supernatant and allowed to react for 5 minutes, followed by centrifugation at 16,000 rpm for 10 minutes. The precipitated pellets were washed 3 times with 70% alcohol. Finally, after drying for 4 hours, the product was diluted in 50 µL of purified water to obtain an mRNA sample.

The obtained sample consisted of double stranded DNA (cDNA) synthesized using PrimeScript™ RT master mix (Takara, Japan). 5 µL of 5X PrimeScript™ RT master mix was added to 20 µL of the mRNA sample, and heated at 37 °C for 20 minutes and then at 85 °C for 10 seconds, thereby obtaining the synthesized DNA double helix structure.

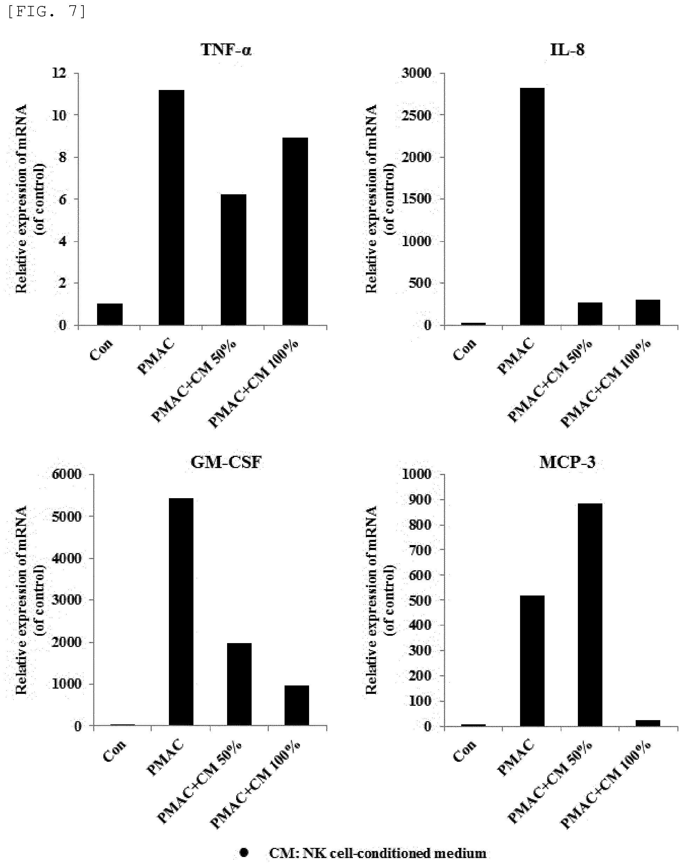

Specific genes were amplified using CFX-96 (Bio-Rad, USA). PCR conditions used for amplification of all genes include: 95 °C for 10 minutes; 95 °C for 30 seconds; and 60 °C for 15 seconds, which were executed by 40 cycles. Expression data were calculated from a cycle threshold (Ct) value using ΔCt according to a quantification method. Further, standardization was conducted using GAPDH. The resultant data were indicated as relative values, compared to the control. Result thereof are shown in FIG. 7.

As shown in FIG. 7, it could be seen that mRNA levels of proinflammatory cytokines such as TNF-alpha, IL-8, GM-CSF and MCP3 were significantly increased in the PMAC treated groups. This means that cell treatment using PMAC may induce degranulation and finally increase the expression of inflammatory cytokines, as compared to a population in normal conditions. However, the results showed that NK-CM significantly reduced the expression of three species, that is, TNF-alpha, IL-8 and GM-CMF proinflammatory cytokines in the 50% or 100% NK-CM treated group. In addition, it could be seen that the expression level of MCP3 was not decreased by 50% treatment but greatly decreased by 100% treatment as a high concentration. This suggests that NK-CM may inhibit the expression of proinflammatory cytokines due to PMAC treatment, and consequently, NK-CM may suppress inflammatory response due to degranulation of HMC-1, thereby having anti-inflammatory effects.

### Example 8. Analysis of NK-CM components

In order to analyze components of NK conditioned-media (NK-CM), analysis was performed using Human Growth Factor Antibody Array C1, human cytokine array C5, and human chemokine array C1 kit (Raybiotech, USA).

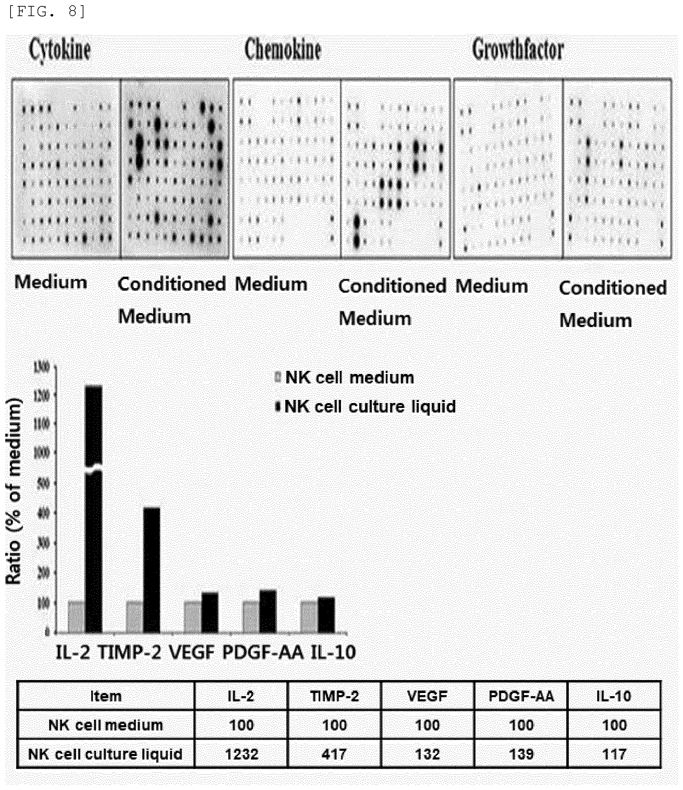

NK cell medium and NK-CM were collected and then analyzed according to the protocol description method of each product provided by Raybiotech. Based on microarray results, the analyzed results are shown with graphs in FIG. 8.

As shown in FIG. 8, it could be seen that IL-2 and IL-10, which are factors associated with atopic dermatitis, were increased in NK-CM, as compared to NK culture liquid. Further, PDGF-AA and VEGF, which are factors associated with hair growth, were increased, and an expression level of TIMP-2, which is a factor associated with wound restoration, was improved. This means that, since items related to atopic dermatitis, hair growth and skin wrinkles are increased in NK-CM including different factors, which are excreted into a medium by NK cells through growth and proliferation, NK-CM can be helpful for improving anti-atopy, hair loss and wrinkles.

## Claims

1. A cosmetic composition for improving atopic dermatitis, hair loss, wounds or skin wrinkles, comprising secretions of natural killer cells.

2. The composition according to claim 1, wherein the natural killer cell secretion includes at least one selected from the group consisting of IL-2, TIMP2, VEGF, PDGF-AA and IL-10.

3. The composition according to claim 1, wherein the natural killer cell secretion is obtained from a residual culture liquid remaining after culturing natural killer cells and then separating and purifying the same.

4. The composition according to claim 3, wherein the culturing includes culturing seed cells in a medium containing feeder cells, a feeder cell stimulating factor and a growth factor.

5. The composition according to claim 4, wherein the seed cells are human-derived cells with a potential to differentiate into natural killer cells.

6. The composition according to claim 5, wherein the human-derived cell is selected from the group consisting of peripheral blood, peripheral blood leukocyte cells, peripheral blood mononuclear cells (PBMC), enriched natural killer cells, isolated natural killer cells, cord blood, hematopoietic stem cells and induced pluripotent stem cells.

7. The composition according to claim 4, wherein the growth factor is interleukin.

8. A pharmaceutical composition for improving atopic dermatitis, hair loss, wounds or skin wrinkles, comprising secretions of natural killer cells.

9. The composition according to claim 8, wherein the natural killer cell secretion includes at least one selected from the group consisting of IL-2, TIMP2, VEGF, PDGF-AA and IL-10.

10. The composition according to claim 8, wherein the natural killer cell secretion is obtained from a residual culture liquid remaining after culturing natural killer cells and then separating and purifying the same.

11. The composition according to claim 10, wherein the culturing includes culturing seed cells in a medium containing feeder cells, a feeder cell stimulating factor and a growth factor.

12. The composition according to claim 11, wherein the seed cells are human-derived cells with a potential to differentiate into natural killer cells.

13. The composition according to claim 12, wherein the human-derived cell is selected from the group consisting of peripheral blood, peripheral blood leukocyte cells, peripheral blood mononuclear cells (PBMC), enriched natural killer cells, isolated natural killer cells, cord blood, hematopoietic stem cells and induced pluripotent stem cells.

14. The composition according to claim 11, wherein the growth factor is interleukin.
